# EUROPÄISCHE PATENTSCHRIFT

(11) **EP 0 781 747 B1**
(45) Veröffentlichungstag und Bekanntmachung des Hinweises auf die Patenterteilung: **01.09.1999**
(21) Anmeldenummer: 97103815.3
(22) Anmeldetag: 07.01.1994
(51) Int. Cl.: C07C 17/20, C07C 25/13

(54) **Verfahren zur Herstellung von 2,3,4,5-Tetrafluor-benzotrifluorid**
Process for the preparation of 2,3,4,5-tetrafluoro-benzotrifluoride
Procédé pour la préparation de 2,3,4,5-tétrafluoro-trifluorométhylbenzène

(30) Priorität: 19.01.1993 DE 4301247
(43) Veröffentlichungstag der Anmeldung: 02.07.1997
(62) Teilanmeldung aus: 94100203.2
(73) Patentinhaber: BAYER AG, 51368 Leverkusen (DE)
(72) Erfinder: Marhold, Albrecht, Dr., 51373 Leverkusen (DE); Andres, Peter, Dr., 40764 Langenfeld (DE)

(56) Entgegenhaltungen:
- EP-A- 0 354 444
- BE-A- 659 239
- PATENT ABSTRACTS OF JAPAN vol. 013, no. 187 (C-592), 2.Mai 1989 & JP 01 013037 A (IHARA CHEM IND CO LTD), 17.Januar 1989,
- Beilstein registry No. 1973474

## Beschreibung

Die Erfindung betrifft ein Verfahren zur Herstellung von 2,3,4,5-Tetrafluorbenzotrifluorid (I) aus den entsprechenden kernchlorierten Benzotrifluoriden durch Umsetzung mit Kaliumfluorid.

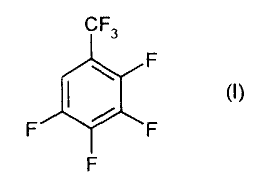

Gemäß DE-OS 3 420 796 kann 2,3,4,5-Tetrafluorbenzoylfluorid aus 2,3,4,5-Tetrachlorbenzoylfluorid durch Umsetzung mit Kaliumfluorid erhalten werden; die Ausbeute beträgt aber lediglich 10 %.

2,3,4,5-Tetrafluorbenzotrifluorid ist Gegenstand der Beilstein Registry Number 1 973 474. Dort wird auf drei mögliche Herstellungsverfahren verwiesen. Gemäß dem ersten Verfahren wird 1 -Chlor-tetrafluor-2-trifluormethylbenzol mit Wasserstoff an Pd-auf-Kohle dehydrochloriert. Beim zweiten Verfahren wird 2,3,4,5-Tetrafluorbenzoesäure mit HF und SF₄ bei 165°C 22 Stunden lang fluoriert. Das dritte Verfahren geht aus von Octafluorcyclohexadien, das mit Trifluormethylacetylen zu einer bicyclischen Zwischenverbindung umgesetzt wird, aus der man durch Umsetzung mit Tetrafluorethylen 2,3,4,5-Tetrafluorbenzotrifluorid erhalten kann. Diese Verfahren sind umständlich, erfordern schwierig zugängliche Ausgangsmaterialien und Reagenzien und deshalb nur von wissenschaftlichem Interesse.

Aus JP 01 013 037 ist die Umsetzung von halogenierten Benzolderivaten mit Kaliumfluorid und einem 2-Komponenten-Katalysator bekannt. Wendet man nur eine Katalysatorkomponente an, werden schlechte Ergebnisse erzielt.

Aus BE 659 239 ist Umsetzung von halogenierten Benzotrihalogeniden mit KF ohne Katalysatorzugabe bekannt. Es werden ebenfalls nur schlechte Ergebnisse erhalten.

Gegenstand der Erfindung ist ein Verfahren zur Herstellung von 2,3,4,5-Tetrafluorbenzotrifluorid 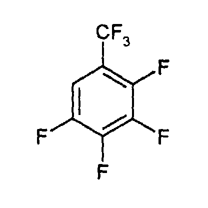 aus Verbindungen der Formel 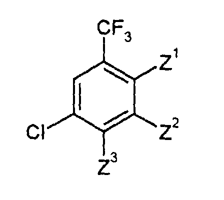 worin
z¹, z², z³ unabhängig voneinander für Fluor oder Chlor stehen,
durch Umsetzung mit Kaliumfluorid in Gegenwart eines Tetraphenylphosphonium- oder Methyltriphenylphosphoniumhalogenids, vorzugsweise -fluorids, - chlorids oder -bromids und in Abwesenheit eines Kronenethers und/oder Polyalkylenglykols.

Die Menge des einzusetzenden Kaliumfluorids richtet sich nach der Anzahl der auszutauschenden Chloratome. Pro val Chlor wird mindestens ein Mol KF eingesetzt, im allgemeinen jedoch 1,1 bis 1,5 Mol. Maximal werden 2 Mol KF/val Chlor verwendet; darüber hinaus ist die Menge KF praktisch ohne Einfluß auf den Fluorierungsgrad und das Verfahren wird unökononmisch.

Als Lösungsmittel bei der Kernfluorierung können die für Fluorierungsreaktionen bekannten inerten Lösungsmittel, z.B. Dimethylformamid, Dimethylsulfoxid, N-Methylpyrrolidon, Dimethylsulfon, Tetramethylensulfon u.a. verwendet werden. Besonders bevorzugt wird jedoch Tetramethylensulfon (Sulfolan) eingesetzt.

Die Reaktionstemperatur liegt zwischen 160 und 260°C in Abhängigkeit vom gewünschten Fluorierungsgrad. Während bei der niederen Temperatur auch noch kernchlorhaltiges Produkt gefunden wird, entsteht bei den höheren Temperaturen bereits ein recht deutlicher Anteil des bekannten 2,3,4,5-Tetrafluorbenzotrifluorids.

Tri-/Tetrachlorbenzotrifluoride können durch Chlorieren von beispielsweise 4-Chlorbenzotrifluorid einfach und in hohen Ausbeuten erhalten werden, während dagegen die Chlorierung von Benzoylchlorid oder 4-Chlorbenzoylchlorid zu zahlreichen, die Ausbeute mindernden Nebenprodukten führt.

### Beispiele

**1. 2,3,4,5-Tetrachlor-benzotrifluorid (Ausgangsverbindung)**
   In einer Ruhrapparatur mit Gasableitung zu einem Vernichtungsturm werden 1 kg 4-Chlorbenzotrifluorid vorgelegt und 15 g gepulvertes Eisensulfid zugegeben. Dann wird Chlor bei 60°C eingeleitet und im Maße der Chloraufnahme die Temperatur gesteigtert, bis der Endpunkt von 110°C erreicht ist. Der Endpunkt wird per GC-Analyse bestimmt. Die Destillation liefert nach einem Vorlauf niederchlorierter Benzotrifluoride 894 g 2,3,4,5-Tetrachlor-benzotrifluorid mit einem Siedebereich von 112 bis 115°C/20 mbar.
**2. 2,3,4,5-Tetrafluor-benzotrifluorid**
   a) In einer Rührapparatur aus V₄A-Stahl werden 835 g (14,4 Mol) Kaliumfluorid in 1440 ml Tetramethylensulfon vorgelegt und bei einem Druck von 20 mbar Lösungsmittel zur Trocknung abdestilliert (100 ml). Dann werden 852 g (3 mol) 2-H-Tetrachlorbenzotrifluorid und 42 g Tetraphenylphosphonium-bromid zugegeben. Anschließend wird unter Eigendruck (3,5 bar) für 18 Stunden auf 210°C erhitzt. Nach Ende der Reaktion wird langsam entspannt und das Produkt über einen Kühler in eine gekühlte Vorlage entspannt. Zum Schluß wird bei abgesenktem Druck (bis 20 mbar) restliches Produkt bis zum Siedepunkt des Lösungsmittels abdestilliert. Es fallen insgesamt 538 g Destillats an, die nach gaschromatographischer Analyse 407 g ≅ 62,2 % Ausbeute an 2-H-Tetrafluorbenzotrifluorid und 126 g (17,9 % Ausbeute) 5-Chlor-2,3,4-trifluorbenzotrifluorid enthalten.
   b) Der Ansatz wird wie unter a) durchgeführt nur mit dem Unterschied, daß vor dem Aufheizen 3 bar Stickstoff aufgedrückt werden und sich ein Gesamtdruck von 6 bar bei 210°C einstellt. Die Aufarbeitung liefert 585 g Grobdestillat, das 400 g (61,1 %) 2-H-Tetrafluorbenzotrifluorid und 170 g 5-Chlor-2,3,4-trifluorbenzotrifluorid enthalt. Die Wiederfindungsrate beträgt 85,3 % an fluorierten Benzotrifluoriden.
   c) In einer Rührapparatur von V₄A-Stahl werden 812 g (14 Mol) Kaliumfluorid in 1400 ml Tetramethylensulfon bei 20 mbar andestilliert (Destillatmenge: 100 ml). Dann werden 40 g Tetraphenylphosphoniumbromid und eine Mischung aus 795 g (2,8 Mol) 2-H-Tetrachlorbenzotrifluorid und 170 g (0,78 Mol) 5-Chlor-2,3,4-trifluorbenzotrifluorid zugegeben und für 18 Stunden unter Eigendruck (max. Druck 3,4 bar) bei 210°C gerührt. Die Destillation liefert 747 g Rohdestillat, das an einer Drehbandkolonne erneut destilliert wird. Es fallen 396 g 2-H-Tetrafluorbenzotrifluorid und 318 g 5-Chlor-2,3,4-trifluor-benzotrifluorid an. Die Wiederfindungsrate der isolierten Ausbeute beträgt 90,5%.
   d) Der Ansatz a) wird wiederholt, wobei sonst gleiche Einsatzmengen bei 200°C an Stelle von 210°C und für 24 Stunden (gegenüber 18 Stunden im Ansatz b) gerührt werden.
      Die destillative Aufarbeitung liefert 596 g Rohdestillat, das 353 g (54 %) 2-H-Tetrafluor-benzotrifluorid und 239 g (34 %) 5-Chlor-2,3,4-trifluorbenzotrifluorid enthält. Die Wiederfindungsrate beträgt 88%.

## Patentansprüche

1. Verfahren zur Herstellung von 2,3,4,5-Tetrafluorbenzotrifluorid aus Verbindungen der Formel 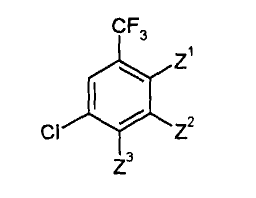 worin
Z¹, Z², Z³ unabhängig voneinander für Fluor oder Chlor stehen,
durch Umsetzung mit Kaliumfluorid in Gegenwart eines Tetraphenylphosphonium- oder Methyltriphenylphosphoniumhalogenids und in Abwesenheit eines Kronenethers und/oder Polyalkylenglykols.

## Claims

1. Process for preparing 2,3,4,5-tetrafluorobenzotrifluoride from compounds of the formula 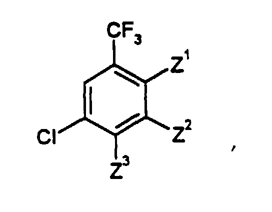 in which
Z¹, Z², Z³ independently of one another represent fluorine or chlorine,
by reaction with potassium fluoride in the presence of a tetraphenylphosphonium or methyltriphenylphosphonium halide and in the absence of a crown ether and/or polyalkylene glycol.

## Revendications

1. Procédé de préparation du 2,3,4,5-tétrafluorobenzotrifluorure à partir de composés de formule 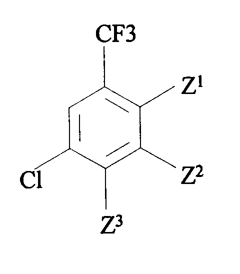 dans laquelle
Z¹, Z², Z³ réprésentent chacun, indépendamment les uns des autres, le fluor ou le chlore, par réaction avec le fluorure de potassium en présence d'un halogénure de tétraphénylphosphonium ou de méthyltriphénylphosphonium et en l'absence d'un éther en couronne et/ou d'un polyalkylèneglycol.
